# EUROPEAN PATENT SPECIFICATION

(11) **EP 3 534 934 B1**
(45) Date of publication and mention of the grant of the patent: **18.05.2022**
(21) Application number: 16920665.3
(22) Date of filing: 03.11.2016
(51) Int. Cl.: A61K 38/46, A61K 38/47, A61K 38/48, A61K 38/54, A61P 21/00, A61P 25/04, A61P 29/00

(54) **ENZYME COMPOSITION FOR THERAPEUTIC MANAGEMENT OF MUSCLE SORENESS**
ENZYMZUSAMMENSETZUNG FÜR DAS THERAPEUTISCHE MANAGEMENT VON MUSKELKATER
COMPOSITION ENZYMATIQUE DE GESTION THÉRAPEUTIQUE DE LA COURBATURE

(43) Date of publication of application: 11.09.2019
(73) Proprietor: Sami Labs Limited, Bangalore 560 058 (IN)
(72) Inventor: MAJEED, Muhammed, East Windsor, NJ 08520 (US); NAGABHUSHANAM, Kalyanam, East Windsor, NJ 08520 (US); MAJEED, Shaheen, East Windsor, NJ 08520 (US)
(74) Representative: Dr. Gassner & Partner mbB
(86) International application number: PCT/US2016/060207
(87) International publication number: WO 2018/084841

(56) References cited:
- US-A- 5 569 458
- US-A1- 2010 189 820
- US-A1- 2011 064 712
- US-A1- 2011 110 905
- US-A1- 2013 323 223
- PAUL C MILLER ET AL: "The effects of protease supplementation on skeletal muscle function and DOMS following downhill running", JOURNAL OF SPORTS SCIENCES, vol. 22, no. 4, 1 April 2004 (2004-04-01), pages 365-372, XP055684135, GB ISSN: 0264-0414, DOI: 10.1080/02640410310001641584
- Kk Vuppala ET AL: "Multi-Enzyme Complex for the Management of Delayed Onset Muscle Soreness after Eccentric Exercise: A Randomized, Double Blind, Placebo Controlled Study", , 1 November 2016 (2016-11-01), XP055684588, Retrieved from the Internet: URL:https://www.omicsonline.org/open-acces s/multienzyme-complex-for-the-management-o f-delayed-onset-musclesoreness-after-eccen tric-exercise-a-randomized-double-blind-pl aceboc-2473-6449-1000113.php?aid=81357 [retrieved on 2020-04-09]
- MAJEED M ET AL: "Multi-Enzyme Complex for the Management of Delayed Onset Muscle Soreness after Eccentric Exercise: A Randomized, Double Blind, Placebo Controlled Study", SPORTS NUTRITION AND THERAPY, vol. 01, no. 03, 11 November 2016 (2016-11-11), XP55552804, DOI: 10.4172/2473-6449.1000113
- UDANI JAY K ET AL: "BounceBack(TM) capsules for reduction of DOMS after eccentric exercise: a randomized, double-blind, placebo-controlled, crossover pilot study", JOURNAL OF THE INTERNATIONAL SOCIETY OF SPORTS NUTRITION, BIOMED CENTRAL LTD, LO, vol. 6, no. 1, 5 June 2009 (2009-06-05), page 14, XP021059194, ISSN: 1550-2783, DOI: 10.1186/1550-2783-6-14
- Anonymous: "Introduction | DigeZyme", , 1 January 2020 (2020-01-01), XP055684652, Retrieved from the Internet: URL:https://www.digezyme.com/digezyme/intr oduction/ [retrieved on 2020-04-09]

## Description

### FIELD OF THE INVENTION

The invention in general relates to enzymes. More specifically the invention relates to use of the digestive enzymes for alleviating the symptoms of delayed on set muscle soreness (DOMS).

### BACKGROUND OF THE INVENTION

### DESCRIPTION OF PRIOR ART

Delayed on set muscle soreness (DOMS) is an exercise induced phenomenon and is a recurrent form of sports injury. It is pressed as a sensation of discomfort, predominantly with the skeletal muscles following unaccustomed physical activity. Associated symptoms include muscle shortening, increased passive stiffness, swelling, decreases in strength and power, localized soreness and disturbed proprioception. This may even cause structural damage to muscle and connective tissue leading to alterations in the muscle function and joint mechanics impairing the performance of athletes and sportsmen.

Treatment strategies for DOMS, both prophylactically and therapeutically, are well known in the art, which include cryotherapy, stretching, anti-inflammatory, drugs, ultrasound, electrical current techniques, homeopathy, massage, compression, hyperbaric oxygen and exercise (Cheung et al., Delayed Onset Muscle Soreness Treatment Strategies and Performance Factors, Sports Medicine 2003; 33 (2): 145-164). Supplementation in the form of branched chain amino acids (Shimomura et al., Branched-Chain Amino Acid Supplementation Before Squat Exercise and Delayed-Onset Muscle Soreness, International Journal of Sport Nutrition and Exercise Metabolism, 2010; 20:236-244) and vitamin C (Connolly et al., The effects of Vitamin C supplementation on symptoms of delayed onset muscle soreness, Journal of Sports Medicine and Physical Fitness, 2006; 46:462-467) are also administered as treatment for DOMS. Beck et al. (Effects of A Protease Supplement On Eccentric Exercise-Induced Markers Of Delayed-Onset Muscle Soreness and Muscle Damage, Journal of Strength Conditioning Research, 2007; 21(3):661-667) tested the validity of a protease based supplement on the markers of DOMS and muscle damage. However, absorption issues and the destruction of enzyme in the guy could limit the effectiveness of traditional anti-DOMS enzyme (protease) therapy. Also, the efficacy of these treatments is limited and inconsistent owing to the lack of understanding surrounding the exact mechanism of DOMS. The avaible treatment modalities are useful in improving some symptoms of DOMS, but not all symptoms are addressed.

It is the principle objective of the present invention to disclose the performance of a comprehensive digestive enzyme blend - DigeZyme^{®} (Protease, Amylase, Lactase, Lipase, Cellulase) as a therapeutic supplement for alleviating the symptoms of DOMS.

The present invention fulfils the aforesaid objective and also provides other related advantages.

### SUMMARY OF THE INVENTION

The present invention discloses a combination of digestive enzymes (Protease, Amylase, Lactase, Lipase, Cellulase) - DigeZyme^{®} for the therapeutic management of delayed onset muscle soreness (DOMS)

Other features and advantages of the present invention will become apparent from the following more detailed description, taken in the conjunction with the accompanying images, which illustrate, by the way of example, the principle of the invention.

### BRIEF DESCRIPTION OF THE DRAWINGS

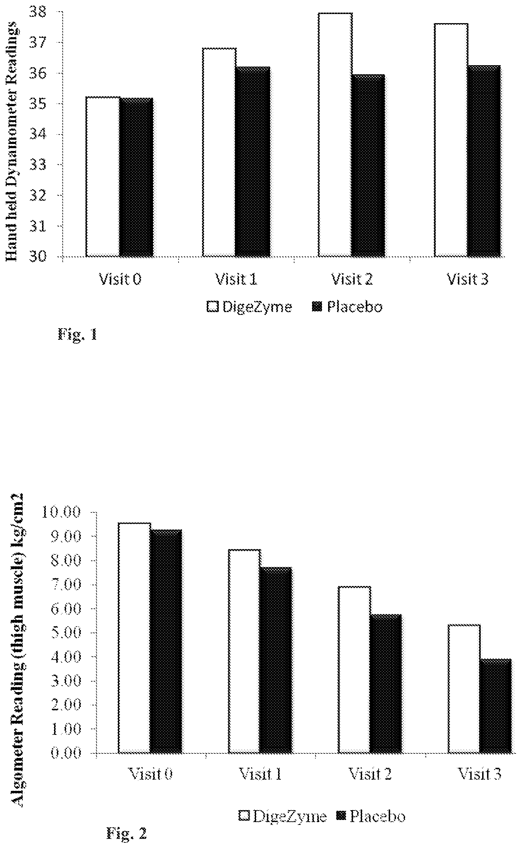

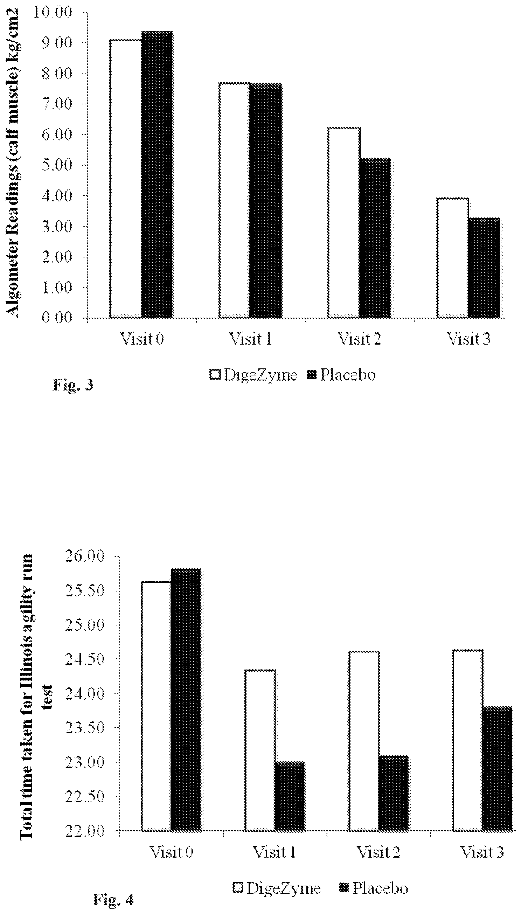

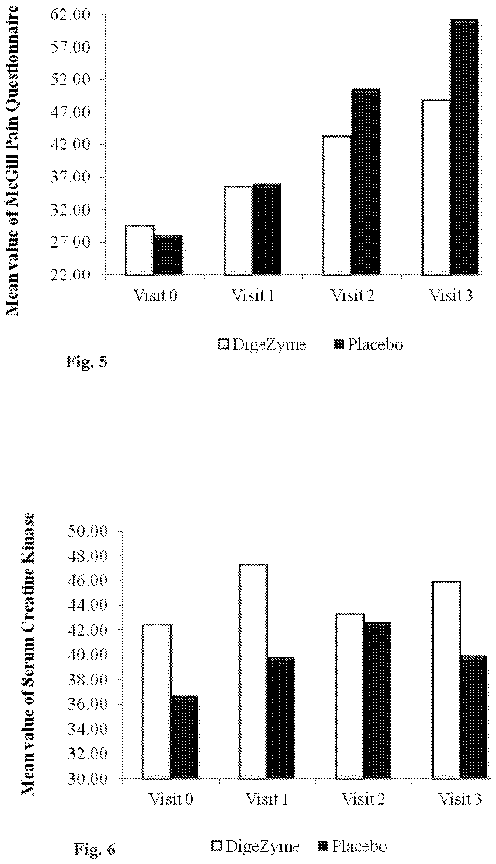

**Fig.1** shows the graphical representation of mean hand held dynamometer readings of test subjects consuming DigeZyme^{®} and placebo during Visits 0-3.
**[Para0010]** **Fig.2** shows the graphical representation of mean Algometer readings in kg/cm² from the thigh muscle of test subjects consuming DigeZyme^{®} and placebo during Visits 0-3.
**[Para0011]** **Fig.3** shows the graphical representation of mean Algometer readings in kg/cm² from the calf muscle of test subjects consuming DigeZyme^{®} and placebo during Visits 0-3.
**[Para0012]** **Fig.4** is the graphical representation of the mean value of total time taken for Illinois Agility agility ru ntest by test subjects consuming DigeZyme^{®} and placebo during Visits 0-3.
**[Para0013]** **Fig.5** is the graphical representation of mean value of McGill pain questionnaire test valued on the test subjects consuming DigeZyme^{®} and placebo supplements during Visits 0-3.
**[Para0014]** **Fig.6** is the graphical representation of mean serum creatinine kinase levels of the test subjects consuming DigeZyme^{®} and placebo during Visits 0-3.
**[Para0015]** **Fig.6** is the graphical representation of mean serum lactate dehydrogenase levels of the test subjects consuming DigeZyme^{®} and placebo during Visits 0-3.

### DESCRIPTION OF THE MOST PREFERRED EMBODIMENTS

In the most preferred embodiment, the present invention discloses a method for the therapeutic management of delayed onset muscle soreness using a comprehensive digestive enzyme blend of Protease, Amylase, Lactase, Lipase and Cellulase (DigeZyme^{®}), said method comprising steps of administering effective concentrations of the enzymes to mammals, presenting with symptoms of delayed onset muscle soreness. In a related embodiment, the individual enzymes in the digestive blend are present in the following concentrations: a) amylase: 24000 DU/g, b) Cellulase: 1100 CU/g, c) Lipase: 200 FIP/g, d) Lactase: 4000 ALU/g and e) Protease: 6000 PC/g. In another related embodiment, the symptoms of delayed onset muscle soreness include pain, tenderness, inflammation, muscle damage and flexibility.

In another preferred embodiement, the invention discloses a composition comprising a comprehensive digestive enzyme blend of Protease, Amylase, Lactase, Lipase and Cellulase (DigeZyme^{®}) for use in therapeutic management of Delayed Onset Muscle Soreness (DOMS).

The specific examples included herein below illustrate the aforesaid most preferred embodiments of the present invention.

### EXAMPLE 1: Study Design

**Product description:** DigeZyme^{®} is an "off - white to creamy white powder of multi-enzyme complex". This Multi-enzyme complex consists of amylase, protease, lipase, cellulase and lactase. Placebo capsules containing equivalent weight of maltodextrin. No differences in colour, taste, texture or packaging were detectable between the two products. Capsules were sealed in identically-appearing, high-density polyethylene bottles with desiccant. The composition of the multi-enzyme complex is given in Table 1.

**Table 1. Composition detail of multi-enzyme complex (DigeZyme) Formulation**

| S. No. | ENZYME | Enzyme activity (Units/g) |
|---|---|---|
| 1 | Alpha-amylase | 24000 DU/g (Dextrinizing Unit /gram) |
| 2 | Cellulase | 1100 CU/g (Cellulase Unit/gram) |
| 3 | Lipase | 200 FlP/g (FédérationInternationalePharmaceutiqueUnit/gram) |
| 4 | Lactase | 4000 ALU/g (Acid Lactase Unit/gram) |
| 5 | Neutral Protease | 6000 PC/g (Protease Unit on L-tyrosine basis/gram) |

| | | |
|---|---|---|
| Enzyme units were defined as per Food Chemical Codex (FCC), 5th ed. 2004. The National Academy Press Washington D.C. | | |

**Ethics approval:** Ethics approval was obtained from Sparsh Hospital for Advanced Surgeries, #146, Infantry Road, Bangalore (registered under central drugs standard control organization as per the gazette notification number F28-10/45-H(1), dated 21 Dec 1945 and last amended vide notification number G.S.R. 76(E) dated 08 FEB 2012.) prior to the initiation of the study.

**Informed consent:** A total of 20 healthy volunteers were included in the study. A written informed consent was obtained from the volunteers during a scheduled initial screening visit. Adequate oral and written information concerning the study was provided to the subjects and subjects were provided with ample opportunity to consider their participation in the study. After obtaining a signed informed consent subjects were screened for the study.

**Study design:** For the DOMS model under study, a prospective, double blind, randomized, and placebo controlled design feature was selected. The analgesic efficacy of DigeZyme^{®} was compared with a matching placebo. The pre specified efficacy end points of the study were assessed on Day 0 (pre exercise) and 72 hours post exercise.

**Participants:** Twenty healthy males with no known musculoskeletal pathology participated in the study. Subjects were excluded if they met one or more of the following exclusion criteria: treatment with anti-inflammatory/analgesic/antioxidant drugs in the previous month, abnormal liver or renal function tests, laboratory findings suggestive of an active inflammatory or infectious process and presence of any known disease. The participants also completed a health history questionnaire designed to identify the degree of risk for cardiovascular or orthopaedic complications during exercise. There were no withdrawals during the course of the study.

**Supplementation with multi enzyme complex:** All participants in the experimental group received the DigeZyme multi enzyme complex supplementation over a 3 day period. During this period, they consumed one capsule of multi enzyme complex thrice a day. The participants in the placebo group received capsules of similar size and colour. They were given identical instructions on dosage of study supplement to be followed. Study supplements were prepared and provided by Sami Labs Limited, Bangalore. The administration of treatment and placebo was blinded for both the participants and the investigator. There were no side effects reported by the participants as a result of the supplementation.

**Data collection protocol:** Subjects visited the clinic on day 0 (baseline visit) and subsequent visits on day 1, day 2 and day 3. After recording vital signs (blood pressure, pulse rate, heart rate and respiratory rate), medical and medication history, physical examination was conducted.

**Randomisation and Allocation concealment:** The randomisation sequence was prepared by an independent statistician, independent of the sponsoring organization and not involved in the conduct or reporting of the study. An alpha numeric code was generated for both the active and placebo to improve the blindness of the study and concealment of allocations. Computer generated random allocation software (version 2.0) was used for the allocation of concealment. Block randomization (only one block) was followed wherein the subjects were randomized to receive either active or placebo. The randomization codes were kept strictly confidential and were accessible only to authorized persons on an emergency basis as per the Sponsor standard operating procedures until the time of unblinding.

**Blinding**: The study was double blinded wherein neither the investigator nor the trial participants knew wheter they would receive the active or the placebo. The investigational products were provided in pre labelled containers to avoid bias.

### EXAMPLE II: Procedures followed:

**Protocol for inducing DOMS**: Subjects were instructed to ingest the study supplement and report to the site after 24-hours for the baseline readings. After 10 hours fasting, heart rate and ratings of perceived exertion were monitored at rest, every 5 minutes during exercise and 10 minutes into recovery. The participants mounted a level motorized treadmill and warmed up for 5 minutes at a self-selected pace. Post 5-min warm up, treadmill speed was iincreased until a heart rate of 80% of predicted maximal heart rate was achieved and were instructed to maintain this pace for 5 min. The treadmill grade at this time was adjusted to 10% and was maintained for 30 minutes. Subjects then completed a 5 minutes active cool-down at a self-selected pace and a 5 minutes seated passive recovery period. Subjects were restricted from indulging in any other physical activity 24 hours prior and 72 hours after the exercise session.

**Quantifying muscle soreness using algometer:** The Muscle Soreness Questionnaires (MSQ) required participants to rate their general soreness on a scale of 1 (normal) to 10 (very, very sore) for the right front thigh and right back thigh (Miller et al., The effects of protease supplementation on skeletal muscle function and DOMS following downhill running. Journal of Sports Sciences 2004; 22:365-372). An algometer was used to quantify muscle pain by applying direct pressure over the muscle. It involves documenting the threshold at which the applied pressure over the muscle is perceived as a sensation of pain rather than a pressure; this is referred to as the pressure pain threshold (PPT). The PPT has been demonstrated to be reliable for measuring pain threshold. The algometer standard is to increase pressure linearly to 5 kg/cm² over 5 seconds according to the method recommended by Fischer. The instrument has a 1 cm² rubber footplate and a scale marked from 2 to 20 kg/cm², in increments of 0.2 kg/cm². Subjects were instructed to report as soon as the sensation of pressure changes to pain by saing 'pain', and 'I will stop.' The footplate of the algometer was held perpendicular to the muscle belly with the gauge turned away from the subject and the examiner. Pressure was increased at a rate of approximately 1 kg/cm²/s until the subject reported 'pain.' The examiner then released the pressure and lifted the algometer off the muscle to read the gauge and record the measurement. The needle on the gauge was returned to baseline before each trial using the pressure release button on the algometer.

**For muscular strength and power using hald held dynamometer:** A Hand-Held Dynamometer (HHD) was used to quantify changes in muscle strength by measuring strength during maximal voluntary isometric contraction (MVC) of the muscle (Kelln et al., Hand-Held Dynamometry: Reliability of Lower Extremity Muscle Testing in Healthy, Physically Active, Young Adults, Journal of Sport Rehabilitation. 2008; 17:160-170). Each participant was asked to perform three repetitions of maximal knee extension and flexion contractions (MVC) at three different positions of knee range of motion (0 degrees, 90 degrees, 120 degrees). The test angles were chosen to account for changes in muscle strength that could occur due to alteration i nthe length of the muscle at different positions of the knee.

**Illinois Agility Run Test:** Agility was measured using Illinois agility run test (Getchell B. Physical Fitness: A Way of Life, 2nd ed. New York: John Wiley and Sons, Inc., 1979). The tests were conducted on a course 10 meters in length and 5 meters in width. Agility time was recorded using a stopwatch. The start, finish and the two turning points were marked with four cones and another four cones were placed down the center equal distance spaced 3.3 meters apart. Subjects were made to lie on their front (head to the start line) and hands by their shoulders. The run started from a standing start on the command 'Go' the stopwatch was started, and the subjects were instructed to get up as quickly as possible and run around the course in the direction indicated without knocking the cones over, to the finish line, at which the timer was stopped.

**Outcome measures:** McGill pain questionnaire comprising of 10 subscales from 0 (no pain) to 10 (worst possible pain) was used to assess pain (Melzack R. The McGill Pain Questionnaire: Major properties and scoring methods. Pain. 1975; 1:277-299; Sullivan et al., An experimental investigation of the relation between catastrophizing and activity intolerance. Pain 2002; 100:47-53). An algometer was used to experimentally induce pain on a predefined point on the patellar tendom five centimetres above the center of the patella, tenderness was assessed. Subjects ranked their pain perception on a scale from 0 to 10. On day 3, assessments were taken at baseline (pre-exercise), post-exercise and further at 24, 48 and 72 hours post-exercise for each arm of the study. Secondary outcomes included assessments of inflammation, muscle damage, flexibility, and the amount of energy expended prior to exercise. Biomarkers creatine kinase and lactate dehydrogenase were monitored pre exercise and 72 hours post exercise. Safety profile of the supplement was assessed by routine haematology, kidney and liver function tests. Adverse events were monitored throughout the study.

**Statistical analysis:** All testing was done using Statistical Analysis Software (SAS) version 9.2. All analyses was conducted using the intent-to-treat population. Patients with no data recorded for a particular parameter were automatically excluded from the analyses of that parameter. For all the data set variables analysis of covariance (ANCOVA) and Wilcox on signed rank sum test were used and the level of significance was set as P < 0,05.

### Example III: Results:

Physical characteristics: The population was essentially healthy without significant confomitant disease or medication intake. Screening characteristics of participants are presented in Table 2.

**Table 2: Subject Demographics Characteristics.**

| **Demographics** | **DigeZyne^{®}** | **Placebo** |
|---|---|---|
| **Height (cm)** | | |
| N | 10 | 10 |
| Mean(SD) | 164.7 (4.99) | 164.2 (5.2) |
| Median | 164.5 | 165 |
| Min, Max | 158,172 | 154,172 |

| **Weight (kg)** | | |
|---|---|---|
| N | 10 | 10 |
| Mean(SD) | 59.96 (4.63) | 59.68 (4.82) |

| **Demographics** | **DigeZyme^{®}** | **Placebo** |
|---|---|---|
| Median | 59.2 | 59.4 |
| Min, Max | 52.5,67 | 51,67 |

| **Body Mass Index (kg/m²)** | | |
|---|---|---|
| | 10 | 10 |
| Mean(SD) | 22.19(1.50) | 22.33 (1.50) |
| Median | 22.3 | 22.3 |
| Min, Max | 20.03,25.39 | 20.03,25.39 |

| **Race** | | |
|---|---|---|
| Central American | 00 | 00 |
| East Asian | 00 | |
| South Asian | 10 | 10 |
| South American | 00 | 00 |
| South East Asian | 00 | 00 |
| Western European | 00 | 00 |
| White | 00 | 00 |

There were no statistically significant difference between subjects in the placebo (n = 10) and the DigeZyme^{®} (n = 10) group. On the day of screening, the mean weight of all the enrolled subjects was 59.3 ± 4.64kgs; mean height was 163.8 ± 4.99 cm and the mean BMI was 22.2 ± 1.50 Kg/m².

Efficacy Evaluation: Delayed Onset Muscle Soreness-quality of life was analyzed throughout the study period as primary efficacy measure. The '*p*' value suggests that there was a statistically significant change in these symptoms from baseline to final visit, between the placebo and active arms. Statistical analysis uuing Analysis of Co-Variance (ANCOVA) showed the primary efficacy parameters were statistically significant (p<0.05) between the multienzyme complex and placebo groups (Table 3).

**Table 3: Efficacy analysis**

| **Subjective Parameters** | | | | | |
|---|---|---|---|---|---|
| **Measure** | **Investigational Products** | | | | **Significanc e** |
| Hand held Dynamometer readings result [grip strength] kg | DigeZyme^{®} | | Placebo Baseline 72 Hour | | 0.4956 |
| | Baseline | 72 Hours Post Exercise | | Post Exercise | |
| | 36.2 (7.86) | 37.6(8.32) | 35.2 (6.84) | 36.2 (5.26) | |
| Thigh muscle (point of pain) Algometer Reading's kg/cm² | 9.5 (0.58) | 5.3 (1.18) | 9.3 (1.03) | 3.9 (1.45) | 0.0436* |
| Calf muscle (point of pain) Algometer Reading's kg/cm² | 9.1 (1.09) | 3.9 (1.20) | 9.4 (1.11) | 3.3 (1.03) | 0.1397 |
| Total Time Taken Illinois Agility Run test (Seconds) | 25.6 (3.43) | 24.6 (4.11) | 25.2 (1.69) | 23.8 (2.91) | 0.7246 |
| The McGill pain questionnaire (total pain score) | 29.5 (2.55) | 48.7 (8.86) | 28.1 (1.45) | 61.3 (7.07) | 0.0061* |

| **Objective Parameters (Serum markers (U/L))** | | | | | |
|---|---|---|---|---|---|
| Serum Creatine Kinase | 42.4 (11.69) | 45.8(10.49) | 36.7 (4.30) | 40.5 (5.42) | 0.5735 |
| Serum Lactate Dehydrogenase | 161.1 (35.56) | 164.2 (31.17) | 162.6 (28.40) | 161.4 (25.44) | 0.2556 |

| | | | | | |
|---|---|---|---|---|---|
| Values expressed as mean (SD) ^{∗}*p value significant (<0.05) between the treatment groups.* | | | | | |

Furthermore, comparative mean vlaues of efficacy assessments between Multi enzyme complex and placebo groups across various visits (baseline, day 1, day 2 and day 3) are presented for efficacy parameters **(****Figs. 1-7****)**. Patients who were on Multi enzyme complex had statistically significant difference for efficacy parameters on day 3 when compared with placebo.

**Pain assessment:** McGill pain questionnaire is a multidimensional pain instrument, the final score is a sum of all the ten individual pain questions. The first nine McGill pain questionnaires were based on assessment of pain (current pain, least pain, and worst pain) while the tenth on the degree of numbness and its interference with function. When differences between multi enzyme complex and placebo were compared, the pain score showed high statistical significance (p=0.0061).

**Tenderness assessment:** On the tenderness quotient, subjects taking multi enzyme complex demonstrated significantly less tenderness, 72 hours after exercise (p = 0.042).

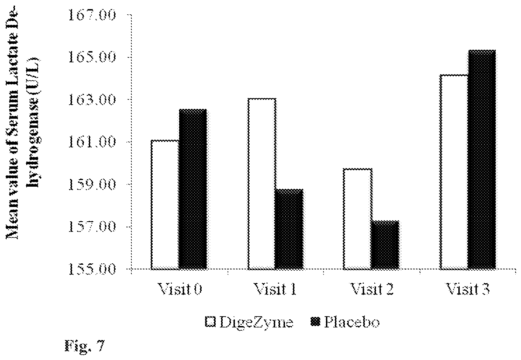

Muscle Damage Assessments: Liberation of biochemical substances such as creatine kinase, lactate dehydrogenase, protein metabolites and myoglobin occurs from muscle cells approximately 24 hours post exercise and have been found in plasma up to 48 hours. Creatine kinase being a surrogate index of muscle damages more indicative of damage or gaps in the sarcolemma. The CK response was less in the multi enzyme complex group suggesting the membrance integrity was maintained to greater extent than the placebo group **(****Fig. 6****)**. 24 hours post exercise, subjects on the placebogroup showed trend towards a higher level of CK than the multi enzyme complex group. This trend continued through the 72 hour assessment. Difference between the multi enzyme complex group and placebo in CK values was not statistically significant. Lactate dehydrogenase also showed a similar phenomenon, which trended higher at 24 and 72 hours post-exercise in the placebo group **(****Fig. 7****).**

**Flexion and Extension Measurements:** On analysing the pre exercise leg flexion measurements, they were found to be equal between the groups for the left leg. Whereas the flexion measurement was found to be significantly greater (p=0.049) for the right leg in the placebo group. When the post exercise flexion measurements were analysed, only the 24-hour right leg flexion measurement was found to be significant (p=0.004) in the multi enzyme complex group.

**Safety Evaluations:** Vital signs such as Blood Pressure, Respiratory Rate, Pulse Rate and any abnormal laboratory parameters were considered for safety evaluations. No clinically significant changes were recorded for descriptive physical examination in both the groups (Multi enzyme complex and placebo). The safety of multi enzyme complex was assessed using adverse event data (occurrence, intensity, and relationship to study drug). No adverse events were noticed in the study.

In the present study, the DigeZyme^{®} multi-enzyme complex capsules demonstrated significant improvement in subjective pain and tenderness, with no significant improvement in levels of markers of inflammation, muscle damage or muscle flexion.

While the invention has been described with reference to a preferred embodiment, it is to be clearly understood by those skilled in the art that the invention is not limited thereto. Rather, the scope of the invention is to be interpreted only in conjunction with the appended claims.

## Claims

1. Digestive enzyme blend comprising protease, amylase, lactase, lipase and cellulase (DigeZyme^{®}) for use in a method for therapeutic management of delayed onset muscle soreness, said method comprising steps of administering effective concentrations of the enzyme blend to mammals presenting with symptoms of delayed onset muscle soreness, wherein the individual enzymes in the digestive blend are present in the concentrations: a) Amylase: 24000 DU/g, b) cellulase: 1100 CU/g, c) lipase: 200 FIP/g, d) lactase: 4000 ALU/g and e) protease: 6000 PC/g.

2. Digestive enzyme blend as in claim 1 for use according to claim 1, wherein the symptoms of delayed onset muscle soreness include pain, tenderness, inflammation, muscle damage and flexibility.

3. Digestive enzyme blend as in claim 1 for use according to claim 1, wherein the mammal is human.

## Patentansprüche

1. Verdauungsenzym-Mischung, umfassend Protease, Amylase, Lactase, Lipase und Cellulase (DigeZyme^{®}) zur Verwendung in einem Verfahren zur therapeutischen Behandlung von verzögert einsetzendem Muskelkater, wobei das Verfahren die Schritte der Verabreichung wirksamer Konzentrationen der Enzym-Mischung an Säuger umfasst, die Symptome von verzögert einsetzendem Muskelkater aufweisen, wobei die einzelnen Enzyme in der Verdauungsmischung in den Konzentrationen a) Amylase: 24000 DU/g, b) Cellulase: 1100 CU/g, c) Lipase: 200 FIP/g, d) Laktase: 4000 ALU/g und e) Protease: 6000 PC/g vorliegen.

2. Verdauungsenzym-Mischung nach Anspruch 1 zur Verwendung gemäß Anspruch 1, wobei die Symptome des verzögert einsetzenden Muskelkaters Schmerzen, Empfindlichkeit, Entzündung, Muskelschädigung und Beweglichkeit einschließen.

3. Verdauungsenzym-Mischung nach Anspruch 1 zur Verwendung nach Anspruch 1, wobei der Säuger ein Mensch ist.

## Revendications

1. Mélange d'enzymes digestives comprenant protéase, amylase, lactase, lipase et cellulase (DigeZyme^{®}) pour utilisation dans un procédé de gestion thérapeutique de la douleur musculaire d'apparition retardée, ledit procédé comprenant des étapes consistant à administrer des concentrations efficaces du mélange d'enzymes à des mammifères présentant des symptômes de douleur musculaire d'apparition retardée, dans lequel les enzymes individuelles dans le mélange digestif sont présentes en les concentrations : a) amylase : 24 000 DU/g, b) cellulase : 1100 CU/g, c) lipase: 200 FIP/g, d) lactase: 4000 ALU/g et e) protéase : 6000 PC/g.

2. Mélange d'enzymes digestives selon la revendication 1 pour utilisation selon la revendication 1, dans lequel les symptômes de douleur musculaire d'apparition retardée incluent la douleur, la sensibilité, l'inflammation, la lésion musculaire et la flexibilité.

3. Mélange d'enzymes digestives selon la revendication 1 pour utilisation selon la revendication 1, dans lequel le mammifère est humain.
